# EUROPEAN PATENT SPECIFICATION

(11) **EP 1 706 127 B1**
(45) Date of publication and mention of the grant of the patent: **20.06.2007**
(21) Application number: 04820047.1
(22) Date of filing: 15.12.2004
(51) Int. Cl.: A61K 36/185

(54) **PENETRATION ENHANCING AGENT AND METHOD OF ITS PRODUCTION FROM THE HEMP SEEDS**
PENETRATIONSVERBESSERNDES MITTEL UND VERFAHREN ZU SEINER HERSTELLUNG AUS HANFSAMEN
AGENT FACILITANT LA PENETRATION ET PROCEDE DE PRODUCTION CORRESPONDANT A PARTIR DE GRAINES DE CHANVRE

(30) Priority: 15.12.2003 CZ 20033412
(43) Date of publication of application: 04.10.2006
(73) Proprietor: Parenteral, A.S., 500 11 Hradec Králové (CZ)
(72) Inventor: HORSKA, Irena, 412 00 Liberec (CZ)
(74) Representative: Fischer, Michael
(86) International application number: PCT/CZ2004/000086
(87) International publication number: WO 2005/056030

(56) References cited:
- WO-A-95/31176
- DE-A1- 3 542 932
- DE-A1- 10 041 700
- GB-A- 1 356 749
- US-B1- 6 307 077
- DATABASE BIOSIS [Online] BIOSCIENCES INFORMATION SERVICE, PHILADELPHIA, PA, US; May 2003 (2003-05), PARKER T D ET AL: "Fatty acid composition and oxidative stability of cold-pressed edible seed oils." XP002320437 Database accession no. PREV200300341311 & JOURNAL OF FOOD SCIENCE, vol. 68, no. 4, May 2003 (2003-05), pages 1240-1243, ISSN: 0022-1147
- SPEISER K ET AL: "HEMP SEED OIL THE WONDER OIL OF THE NEW MILLENNIUM" HAPPI HOUSEHOLD AND PERSONAL PRODUCTS INDUSTRY, DORLAND PUBLISHING, DENVILLE, NJ, US, vol. 36, no. 6, June 1999 (1999-06), pages 106-109, XP000829895 ISSN: 0090-8878

## Description

### Field of technology

The invention pertains to a conveying and absorption agent, preferably for the active substances of the pharmaceutical, dietetic, and cosmetic preparations. The invention further relates to a method of production of the conveying and absorption agent from the hemp seeds.

### Background of the invention

The pharmaceutical, dietetic, and cosmetic preparations rely on crude-oil, organic, or vegetable basis. The preparations that rest on the crude oil base, made up e.g. by a Vaseline or paraffin have a limited capacity to penetrate into the skin deeper layer. In order to enhance the capacity to penetrate into the deeper skin layers the organic-chemical conveyors or keratolythics and emulsifiers of a synthetic, unnatural origin are therefore preferably being added in particular into the ointment and cream bases. The ointment and cream bases originating in crude oil are not much able to bind upon themselves the active substances. The synthetic absorbents that are, however, unable to bind upon themselves the modern bioactive substances such as cytokines, CD monoclonal antibodies, derivates of arachid acid, low-molecular peptides, modern antibiotics, etc. are therefore used to convey the active substances into the deeper skin layers. Further disadvantage of the ointment and cream bases, having originated in crude oil, is the fact that they have no therapeutic effect and that the synthetic organic-chemical conveyors, emulsifiers, and conserving substances tend to evoke allergic and other dermal reactions. The ointment and cream bases of animal or vegetal origin used until now contain the aliphatic acids and fats that are relatively unstable, tend to oxidize, and - as such - presume the use of the synthetic organic-chemical stabilizers with the relatively high levels of concentration, evoking adverse dermal responses. Although the ointment and cream bases of animal or vegetal origin are also capable to penetrate into the deeper skin layers, they have, however, a very limited capacity to bind upon themselves the bioactive substances such as the cytkins, CD monoclonal antibodies, arachid acid derivates low-molecular peptides, modern antibiotics, etc., and cannot therefore convey efficiently the active substances into the deeper skin layers. The ointment and cream bases of an animal origin, fats, lard, sheep wool fats, sperm whale stuff, and bee wax, as well as the ointment and cream bases of vegetal origin, helianthi oleum, arachidis oleum types, lino oleum, amygdalae oleum, ricini oleum oil types, herbal oils are being combined, preferably by mixing them together, but these combined bases neither remove the necessity to use the organic-chemical conveyors, emulsifiers, and conserving substances of a synthetic, non-natural origin nor have any better capacity to bind upon themselves and convey the bioactive substances into the deeper skin layers.

A dermal inflammation healing agent, using the oil, cold-pressed out of the hemp seed, as an active substance is known from the specification DE 10041700. This dermal inflammation healing agent can, in accordance with the specification DE 10041700, be used in an emulsion with emulsifier such as Lizitin, for application onto the inflamed portions of skin. But the inflammation healing agent as per Volume DE 10041700 does not have any satisfactory effect and its applicability period/shelf life is short. When used, the preparation presumes a simultaneous application of the adversely acting synthetic stabilizers and emulsifiers. A cosmetic and therapeutic agent, composed of the hemp oil which is relieved of the oxidation-prone additives that are degrading this non-refined hemp oil via a bacterial biodegradation and that are evoking both the chemical and pharmacological instability, is known from the Volume CZ - U1-13335. When improved like this, the hemp oil can better counteract the action of the UV radiation and has an enhanced capacity to bind upon itself other pharmaceutics. Production of the cosmetic and therapeutic agent per Volume CZ - U1-13335 consists of multiple stages, being very demanding in technological terms, and its manufacturing cycle is very long. Removal of the oxidation-prone additives is incomplete.

Document GB 1356749 is known, which discloses a method of producing vegetable fats and oils by extraction with a solvent, therein the fat or oil is removed from the vegetable matter by extraction with supercritical gases, comprising carbone dioxide. The use of carbone dioxide is based on the inactivity of the inert gas in the respect of the taste and health. With the method according to the document GB 1356749 it is possible for the fats or oils to be completely extracted in their natural composition from the vegetable metrial without chemical modification even of the residue. The document GB 1356749 does not disclose any other quality or feature of the disclosed method. Document DE 3542932 is known, which discloses a method of a soft extraction of crushed up oil seeds with carbone dioxide. The disclosed method is used due to time efficiency and gentleness in respect to the oil seeds. The document DE 3542932 does not disclose any other quality or feature of the disclosed method.

Object of the invention is to avoid the disadvantages of the state of the art as mentioned above and to provide a natural, more efficient active substance conveyor and absorbent, possessing moreover a better capacity to bind upon itself the bioactive substances, to penetrate into the deeper skin layers, and to convey the bioactive substances without the use of any synthetic carriers, featuring a longer utility period without the necessity to use any conservation chemicals, which - in its primary composition - would also show - without any additives - healing and relaxing effects, preferably in case of the dermal diseases and also contain - in its primary composition - a natural UV protective factor.

Furthermore, the objective of the invention is to provide the method of production of hemp seed oil which - in respect to the state of the art as mentioned above - would better penetrate and convey the active substances into the deeper skin layers without the use of any synthetic carriers and would bind upon itself a broader spectrum of the bioactive aubstances. A further objective is to produce an agent which with these properties easily and reliably in the industrial conditions.

### Subject-matter of the Invention

To a major extent the disadvantages of the current state of technology are eliminated and the invention objectives achieved by a hemp seed oil for transfer and absorption of active substances of pharmaceutical and cosmetic preparations into the skin according to the invention consisting in that it is a product of extraction of the milled down hemp seeds by means of carbon dioxide. Advantageously one weight share of the extract may create a mixture with two weight shares of the solution containing up to 30 % in weight of sodium bicarbonate. The invention objectives are also achiebed by a method of production of hemp seed oil for transfer and absorption of active substances of pharmaceutical and cosmetic preparations into the skin, consiting in that the hemp seeds are milled down to hemp flour, then pressure-extracted by means of the carbon dioxide to hemp oil. Advantageously, hemp seeds may be milled down to a fine hemp flour, the milled down hemp flour then poured into the extraction cartridges that are then inserted into the extractor; the extractor gets closed and carbon dioxide is driven into it at the temperature between about 35 °C - 45 °C and under pressure between 25 MPa - 35 MPa, advantageously at 40 °C and the pressure 20 Mpa, with the hemp oil extraction process slowed down the carbon dioxide pressure in the extractor is reduced down to the value of the ambient atmospheric pressure and the hemp oil is separated from the carbon dioxide, then the carbon dioxide is taken out of the extractor to a reserve tank and stored there in its supercritical condition. Advantageously, 2 % - 35 % in weight of the crushed silicon sand may be mixed into the extracted hemp oil for removal of the chlorophyll and waxes; following the surface absorption, the crushed silicon sand is filtered out. According to the invention the hamp seed oil manufactured by a method according to the invented method of production may be used for transfer and absorption of active substances of pharmaceutical and cosmetic preparations into the skin.

The cosmetic, dietetic, and therapeutic preparations containing a conveying and absorption agent for conveying the active substances according to the invention have better efficiency as the conveying and absorption agent has a strong capacity to bind upon itself the bioactive substances. Unlike the agents known until now, the conveying and absorption agent according to the invention is able to bind upon itself a broader spectrum of the bioactive substances such as e.g. cytokines, CD monoclonal antibodies, arachid acid derivates, low-molecular peptides, modern antibiotics, and other substances, to penetrate and convey them into the deeper skin layers without the use of any synthetic carriers. The conveying and absorption agent according to the invention does not irritate skin, but on the contrary has more bio-chemical qualities, is healing eczema, acne, and relaxes notably psoriasis without application of any other active substances e.g. tar, hormonal substantia, contains a natural UV filter and a minimum of the substances capable of oxidation or reduction. Cosmetic and therapeutic preparations that contain the conveying and absorption agent according to the invention are stable for the period of at least two years without the necessity to apply any chemical conserving substances. The cosmetic and therapeutic preparation that contains the conveying and absorption agent according to the invention features a high concentration of linoleic acid, linolenic acid, gamma-linolenic acid, prostaglandins, prostacyclines, tromboxanes, leucotriens, and high level of purity, as it almost fails to contain the substances from the group of proteins, amino acids, and sugars. Major advantage rests in the fact, that when combined with the crude oil, animal, and other vegetal bases, the conveying and absorption agent according to the invention does not lose its efficiency and retains all the above-mentioned characteristics. An advantage of the method of production of the conveying and absorption agent according to the invention and the cosmetic and therapeutic preparations that contain the conveying and absorption agent according to the invention rests in its efficiency given by the fact that the above-mentioned bioactive substances that - thanks to their effects - influence the cellular nutrition in the skin bottom layers, making thus the therapy more effective, are remaining in the hemp oil. The conveying and absorption agent according to the invention also manifested itself as having positive impacts on the human health as a food ingredient as well.

### Examples of execution

The following examples demonstrate usage of the hemp oil based conveying and absorption agent according to the invention for production of the ointment and cream bases.

### Example 1

Cosmetic and therapeutic preparation based on the hemp oil and consisting of 0.5 % in weight of the hemp oil extracted from the hemp seeds by means of carbon dioxide, 84.5 % in weight of the Flava Vaseline, and 15 % in weight of the propolis relieved of its pollen share.

### Example 2

Cosmetic and therapeutic preparation based on the hemp oil and consisting of 5 % in weight of the hemp oil extracted from the hemp seeds by means of carbon dioxide, 80.0 % in weight of the lard, and 15% in weight of propolis relieved of its pollen share.

### Example 3

Cosmetic and therapeutic preparation based on the hemp oil and consisting of 20 % in weight of the hemp oil extracted from the hemp seeds by means of carbon dioxide, 50 % in weight of hard paraffin, and 30% in weight of propolis relieved of its pollen share.

### Example 4

Cosmetic and therapeutic preparation based on the hemp oil and consisting of 50 % in weight of the hemp oil extracted from the hemp seeds by means of carbon dioxide, 40 % in weight of yellow wax, and 10% in weight of ricini oleum.

### Example 5

Cosmetic and therapeutic preparation based on the hemp oil and consisting of 70 % in weight of the hemp oil extracted from the hemp seeds by means of carbon dioxide, 20 % in weight of white wax, 5 % in weight of arachidis oleum, and 5 % in weight of propolis relieved of its pollen share.

### Example 6

Therapeutic preparation based on the hemp oil and consisting of 95 % in weight of the hemp oil extracted from the hemp seeds by means of carbon dioxide, 4 % in weight of the sheep wool, and 1 % in weight of propolis relieved of its pollen share.

### Example 7

Therapeutic preparation based on the hemp oil and consisting of 99.5 % in weight of the hemp oil extracted from the hemp seeds by means of carbon dioxide, and 0.5 % in weight of propolis relieved of its pollen share.

### Example 8

Therapeutic and dietetic preparation in the form of capsules as a food ingredient based on the hemp oil and consisting of 100 % in weight of the hemp oil extracted from the hemp seeds by means of carbon dioxide.

### Example 9

Therapeutic preparation based on the hemp oil and consisting of 33.33 % in weight of the hemp oil extracted from the hemp seeds by means of carbon dioxide, 40 % in weight of amygdalea oleum, 20 % in weight of sodium bicarbonate, and 6.66 % in weight of propolis relieved of its pollen share.

### Example 10

Cosmetic and therapeutic preparation based on the hemp oil and consisting of the mixture of 33.33 % in weight of the hemp oil extracted from the hemp seeds by means of carbon dioxide, 50 % in weight of helianthi oleum and 10 % in weight of sodium bicarbonate, and 6.66 % in weight of propolis relieved of its pollen share.

According to the method of production of the conveying and absorption agent according to the invention, the hemp seeds are ground down to fine-grain flour which is then poured into the extraction cartridges that are then inserted into the extractor. The extractor is filled with the extraction cartridges with hemp flour and closed. Then, carbon dioxide is driven into the extractor at the temperature of 30°C - 45 °C and pressure of 25 MPa - 35 MPa, advantageously at 40 °C and pressure 29 MPa. Within the scope of the above temperature and pressure values, the method of production is highly efficient, but this efficiency tends to drop sizably beyond this scope. By virtue of the carbon dioxide pressure the hemp oil gets extracted from the hemp flour. With the hemp oil extraction slowed down the carbon dioxide pressure in the extractor falls down to the value of the ambient atmospheric pressure and the hemp oil gets separated from the carbon dioxide. Then, the carbon dioxide is fully drawn out of the extractor and led into the reserve tank where it is then being stored in its supercritical condition. During the extraction, the carbon dioxide action upon the hemp flour is quite inert, not causing any chemical reactions. No reactions take place during the extraction; no new chemical bonds arise in the components involved in the extraction. Carbon dioxide is inert to proteins, sugars, and starches. In comparison with method of hemp oil production by cold pressing of the hemp seeds, there is no need to refine the hemp oil when extracted from the ground hemp seeds by means of the carbon dioxide or to subject it to any other modifications. Preferably the gamma-linolenic acid and arachid acid, as well as other substances will remain well preserved in the hemp oil extracted. Where a more transparent, lighter-shaded oil has to be gained and where the chlorophyll and waxes have to be removed, the filtration can advantageously be carried out by means of an inert diacomateous earth - a special sand, which (making use of the physical processes) binds upon itself these unwanted substances. Within this filtration technique, the diacomateous earth is mixed into the hemp oil in the ratio of 2 % - 35 % in weight, being then filtered, advantageously through a multi-cloth filter in which, in order to get the active surface as large as possible, a set of cloth-coated frames, arresting the chlorophyll and wax wrapped diacomateous earth while the oil is poured through, is embedded. The ratio from 2 % - 35 % in weight of the crushed silicon sand in the hemp oil appeared advantageous in respect of the efficient absorption of the unwanted substances on the surfaces of the crushed silicon sand. Advantageously, this proportion can be narrowed to 5 % - 20 % in weight, when a spontaneous surface absorption takes place. When obtained by extraction, the hemp oil is showing the following typical parameters:
density 0.910 - 0.926 g/cm3
refraction index 1.472 - 1.480
peroxide number ≤ 15 02/kg
number of acidity > 25.0 mg KOH/g
iodine number 150 - 170 g as I2/100

When extracted with carbon dioxide, hemp oil contains in high concentration the substances from the group of linoleic acid, linolenic acid, gamma-linolenic acid, dihomogamalinolene acid, arachidine acide, prostaglandins, prostacyclines, tromboxanes, leucotriens and reaches a high level of purity, as it is quite disposed of the substances from the group of proteins, amino acids, and sugars.

### Industrial use

According to the invention, the conveying and absorption agent can be used as a part of the cosmetic, pharmaceutical, and dietetic preparation's bases, preferably as a part of the bases of the ointment and cream preparations being applied in order to relax, cure eczema, acne, and for substantial relaxing of psoriasis, as a drug and as a part of an ointment base for the subsequent mixing in of various substances, extracts, bioactive substances, pharmaceutically recognized substances, and as a component in the cosmetics, preferably in the suntan creams and skin regenerating creams, as well as in production of all other skin-friendly cosmetic preparations, such as soap, shampoo, gel, pharmaceutical and dietetic preparations.

## Claims

1. Hemp seed oil for transfer and absorption of active substances of pharmaceutical and cosmetic preparations into the skin,
**characterised by that**
it is a product of extraction of the milled down hemp seeds by means of carbon dioxide.

2. Hemp seed oil according to the claim 1,
**characterised by that**
one weight share of the extract creates a mixture with two weight shares of the solution containing up to 30 % in weight of sodium bicarbonate.

3. A method of production of hemp seed oil for transfer and absorption of active substances of pharmaceutical and cosmetic preparations into the skin,
**characterised by that**
the hemp seeds are milled down to hemp flour, then pressure-extracted by means of the carbon dioxide to hemp oil.

4. A method of production according to the claim 3,
**characterised by that**
hemp seeds are milled down to a fine hemp flour, the milled down hemp flour is then poured into the extraction cartridges that are then inserted into the extractor; the extractor gets closed-and carbon dioxide is driven into it at the temperature between about 35 °C - 45 °C and under pressure between 25 MPa - 35 MPa, advantageously at 40 °C and the pressure 20 Mpa, with the hemp oil extraction process slowed down the carbon dioxide pressure in the extractor is reduced down to the value of the ambient atmospheric pressure and the hemp oil is separated from the carbon dioxide, then the carbon dioxide is taken out of the extractor to a reserve tank and stored there in its supercritical condition.

5. A method of production according to the claim 3 or 4,
**characterised by that**
2 % - 35 % in weight of the crushed silicon sand is mixed into the extracted hemp oil for removal of the chlorophyll and waxes; following the surface absorption, the crushed silicon sand is filtered out.

6. A use of the hemp seed oil manufactured by a method according to one of the claims 3-5 for transfer and absorption of active substances of pharmaceutical and cosmetic preparations into the skin.

## Patentansprüche

1. Öl aus Hanfsamen zur Beförderung und Absorption von Komponenten pharmazeutischer und kosmetischer Präparate in die Haut,
**dadurch gekennzeichnet, dass**
es ein Produkt einer Kohlendioxid-Extraktion von zermalmten Hanfsamenkörnern ist.

2. Öl aus Hanfsamen laut Anspruch 1,
**dadurch gekennzeichnet, dass**
ein Masseteil des Extrakts ein Gemisch mit zwei Masseteilen einer Lösung bildet, die bis zu 30 Masseprozente Natriumkarbonat enthält.

3. Verfahren zur Herstellung von Öl aus Hanfsamen zur Beförderung und Absorption von Komponenten pharmazeutischer und kosmetischer Präparate in die Haut nach Anspruch 1,
**dadurch gekennzeichnet, dass**
die Hanfsamen dem Zermahlen in Hanfmehl unterzogen werden und das Hanfmehl unter Druck mit Kohlendioxid extrahiert wird.

4. Verfahren zur Herstellung von Öl nach Anspruch 3,
**dadurch gekennzeichnet, dass**
die Hanfsamen in feines Mehl zermahlen werden, das gemahlene Hanfmehl in Extraktionseinlagen geschüttet wird, die in den Extraktor eingebracht werden, der Extraktor verschlossen wird und bei einer Temperatur von etwa 35 - 45°C und einem Druck von 25 - 35 MPa, bevorzugt 40°C und 29 MPa, Kohlendioxid dort einströmt, sich der Druck des Kohlenmonoxids nach der Verlangsamung der Extraktion des Hanföls im Extraktor bis auf den Wert des atmosphärischen Umgebungsdrucks verringert und das Hanföl von dem Kohlendioxid getrennt und nachfolgend das Kohlendioxid vollständig aus dem Extraktor in einen Reservebehälter abgeleitet wird, wo es im superkritischen Zustand lagert.

5. Verfahren zur Herstellung von Öl nach Anspruch 3 oder 4,
**dadurch gekennzeichnet, dass**
zur Entfernung von Chlorophyll und Wachsen in das aus den Hanfsamen extrahierte Öl 2 - 35 Masseprozente Mahlgut aus Siliziumsand beigemischt und das Mahlgut des Siliziumsands nach der Oberflächenabsorption abgefiltert wird.

6. Verwendung von Öl aus Hanfsamen, hergestellt nach einem der Ansprüche 3 bis 5, zur Beförderung und Absorption aktiver Stoffe von pharmazeutischen und kosmetischen Präparaten in die Haut.

## Revendications

1. l'huile de graines de chanvre pour le transport et l'absorption des composantes des préparations pharmaceutiques et cosmétiques dans la peau,
**caractérisée par le fait**
**qu'**elle est le produit d'extraction des graines de chanvre broyées avec du gaz carbonique.

2. L'huile de graines de chanvre conformément à la revendication n°1
**caractérisée par le fait**
**qu'**une fraction de masse de l'extrait forme un mélange avec deux fractions de masse de la solution contenant jusqu'à 30% de masse de carbonate de sodium.

3. Méthode de production d'huile de graines de chanvre pour le transport et l'absorption des composantes des préparations pharmaceutiques et cosmétiques dans la peau, conformément à la revendication n°1,
**caractérisée par le fait**
**que** les graines de chanvre sont soumises au broyage en farine de graines de chanvre et la farine de graines de chanvre est extraite sous pression par du gaz carbonique.

4. Méthode de production d'huile conformément à la revendication n°3,
**caractérisée par le fait**
**que** les graines de chanvre sont broyées en une farine fine, la farine de chanvre fine est introduite dans les pièces d'insertion de l'extracteur, qui sont introduites dans l'extracteur ; on ferme l'extracteur, on y introduit du gaz carbonique à une température comprise entre 35 et 45°C et à une pression comprise entre 25 et 35 MPa, avec une préférence pour 40°C et une pression de 29 MPa ; après ralentissement de l'extraction de l'huile de chanvre, la pression du gaz carbonique dans l'extracteur est réduite jusqu'au niveau de la pression atmosphérique ambiante et l'huile de chanvre est dissociée du gaz carbonique puis le gaz carbonique est éliminé en totalité de l'extracteur dans le récipient de réserve, où il est stocké à l'état supercritique.

5. Méthode de production d'huile conformément à la revendication n°3 ou 4,
**caractérisée par le fait**
**que** pour l'élimination de la chlorophylle et des cires, on mélange dans l'huile de graines de chanvre extraite 2 à 35% en masse de sable silicique broyé et après l'absorption de surface, le sable silicique broyé est filtré.

6. Utilisation de l'huile de graines de chanvre produite conformément à l'une des revendications n°3 à 5 pour le transport et l'absorption des matières actives des préparations pharmaceutiques et cosmétiques dans la peau.
